# EUROPEAN PATENT APPLICATION

(11) **EP 3 933 016 A1**
(43) Date of publication of application: **05.01.2022**
(21) Application number: 20183297.9
(22) Date of filing: 30.06.2020
(51) Int. Cl.: C11B 1/00, C11B 1/02, C11B 1/04, C11B 1/10

(54) **METHOD OF ISOLATING LIPIDS FROM A LIPIDS CONTAINING BIOMASS**

(71) Applicant: Evonik Operations GmbH, 45128 Essen (DE); DSM IP Assets B.V., 6411 TE Heerlen (NL)
(72) Inventor: DIEHL, Michael, Omaha, NE 68102 (US); HEINING, Annika, 63791 Karlstein am Main (DE); HEINING, Martin, 63791 Karlstein am Main (DE); JOHNSON, Michael Benjamin, Columbia, MD 21045 (US); LEBERT, Jochen, 63864 Glattbach (DE); LEININGER, Neil Francis, 4303 Kaiseraugst (CH); TARWADE, Vinod, Ellicott City, MD 21043 (US); TINSLEY, David Allen, Versailles KY 40383 (US)
(74) Representative: Evonik Patent Association

(57) **Abstract**

**Summary**

The present invention relates to a method of isolated polyunsaturated fatty acids containing lipids from a lipids containing biomass.

## Description

The invention relates to a method of isolating polyunsaturated fatty acids containing lipids from a lipids containing biomass.

PUFAs (polyunsaturated fatty acids) containing lipids are of high interest in the feed, food and pharmaceutical industry. Due to overfishing there is a high need for alternative sources for PUFAs containing lipids besides fish oil. Besides certain yeast and algal strains in particular microalgal cells like those of the order Thraustochytriales are a very good source for PUFAs containing lipids.

Previously, the isolation of the oil from the cells was carried out by using organic solvents like hexane. But the use of organic solvents leads to hazardous operating conditions, requires the use of expensive explosion-proof equipment and requires the implementation of an expensive solvent recovery process to avoid pollution of the environment.

Thus, as an alternative way for isolating the oil the salting-out of the oil with high amounts of sodium chloride was developed. But the use of high amounts of sodium chloride leads to a delipidated biomass by-product which due to the high salt content cannot be utilized as a feed ingredient, so that the process is not very sustainable. Further, the high salt concentration leads to fast corrosion of the used steel equipment.

Thus, there was a need to develop a method for efficiently isolating the oil from the biomass which works without the use of organic solvents as well as without the use of high amounts of salt. Such methods are described in WO 2018/011275, WO 2018/011286, WO 2018/013670 and WO 2018/122057. Here it could in particular be shown that if an appropriate amount of base equivalents is added to a biomass suspension containing the PUFAs containing lipid, an efficient separation of the oil from the biomass can be realized without the need to add organic solvents or high amounts of salts.

Basing on this state of the art, the object of the invention was to further improve the efficiency of the oil isolation process.

Surprisingly, it turned out that the overall efficiency of the oil isolation process could be further improved, if before the mechanical separation of the oil phase from the aqueous phase the ratio of oil to total dry matter (TDM) is at least 0.5, preferably in the range of 0.5 to 0.9.

Thus, a first subject of the present invention is a method of isolating a polyunsaturated fatty acids (PUFAs) containing lipid from a biomass, comprising the following steps:
a) Providing a demulsified PUFAs containing composition, characterized in that the ratio of oil to total dry matter (TDM) in the suspension is at least 0.5, preferably 0.5 to 0.9, more preferably 0.5 to 0.7 or 0.5 to 0.65, above all 0.5 to 0.6;
b) Separating the oil containing light phase from the water, salt, remaining oil and cell debris containing heavy phase by mechanical means.

Preferably, the suspension according to (a) has a TDM content of 20 to 60 wt.-%, more preferably of 25 to 55 wt.-%, in particular of 30 to 50 wt.-%. The TDM content can be adjusted, if necessary, by concentrating either the demulsified PUFAs containing composition itself or, preferably, by concentrating the cell containing suspension, before demulsification is carried out.

There are principally two different ways for realizing the subject of the invention.

One way of realizing the invention is to prepare the demulsified PUFAs containing composition, which exhibits a ratio of oil to TDM of at least 0.5, preferably 0.5 to 0.9, by carrying out a demulsification process as principally disclosed in the state of the art, but adding oil in the course of the demulsification process to increase the ratio of oil to TDM in the composition before the mechanical separation of the oil containing phase from the aqueous phase takes place.

Thus, in a preferred embodiment of the invention, the preparation of the demulsified PUFAs containing composition comprises the following steps:
a) Providing a suspension of a biomass comprising cells which contain a PUFAs containing lipid;
b) Lysing the cells of the biomass at least in part;
c) Concentrating the suspension to a total dry matter (TDM) content of 20 to 60 wt.-%, if the suspension has a lower TDM content;
d) Adjusting in the suspension a temperature of 20°C to 100°C, preferably 25, 30, 40, 50 or 60°C to 100°C, more preferably 65°C to 95°C, in particular 70 to 90°C;
e) Keeping the temperature in the range as depicted in (d) for at least 0.5 hours, preferably for at least 1, 2, 3, 4, 5, 6, 7 or 8 hours, more preferably for 1 to 36 hours, in particular for 2, 3, 4, 5, 6, 7 or 8 hours to 36 hours, more preferably 10 to 24 hours, while adding in total 7.5 to 25 moles, preferably 8.5 to 22 moles, in particular 10 to 20, more preferably 11 to 18, above all 12 to 17 moles, of base equivalent to 10 kg of total dry matter as contained in the suspension;
f) Adjusting a pH value of 5.0 to 8.5, preferably 5.0 to 7.5, more preferably 5.0 to 6.5, and incubating for 0.1 to 6 hours, preferably 0.5 to 4 hours;
   characterized in that oil is added to the suspension sometime after step (b), preferably after step (e) or after step (f), more preferably after step (f), so that the ratio of oil to total dry matter (TDM) reaches a value of at least 0.5, preferably 0.5 to 0.9 or 0.5 to 0.8, in particular 0.5 to 0.7 or 0.5 to 0.65, more preferably 0.5 to 0.6.

The oil which is added to increase the ratio of oil to TDM can be any kind of edible oil, in particular a microbial oil, a vegetable oil, an oil of animal origin or mixtures thereof. In a preferred embodiment of the invention, the oil is an oil which contains also PUFAs.

The oil of animal origin is preferably an oil of a marine organism. According to the invention, "oil of a marine organism" or "marine oil" is to be understood in general to mean an oil obtained from a marine organism, preferably from a marine animal. Besides fish oil, which is preferred according to the invention, it also refers to oils isolated from other marine organisms, in particular from marine animals, for example from krill, bivalves, squids or shrimps. Preferably, the marine oil to be used according to the invention is fish oil, in particular a fatty oil from fish, especially preferably a fatty oil from fish of the families Engraulidae, Carangidae, Clupeidae, Osmeridae, Scombridae and/or Ammodytidae.

The use of marine oils, in particular fish oil, and vegetable oils, in particular canola oil and soy oil, turned out to be very suitable for improving the oil separation according to the invention.

As the biomass of the invention is preferably a microbial biomass, in particular a biomass of the class Labyrinthulea, addition of marine oil preferably leads to a product which contains a mixture of a PUFAs containing microbial oil, in particular of the class Labyrinthulea, and marine oil. Thus, a further subject of the invention are oil compositions, comprising microbial oil, in particular of the class Labyrinthulea, preferably of the genera Aurantiochytrium or Schizochytrium, and marine oil, in particular fish oil, in a (w/w) ratio of 30:1 to 1:30, preferably in a (w/w) ratio of 20:1 to 1:20, more preferably in a (w/w) ratio of 10:1 to 1:10, 5:1 to 1:5 or 2:1 to 1:2.

Thus, a further subject of the invention is also a lipid, comprising 5 to 95 wt.-%, in particular 10 to 90 wt.-% or 20 to 80 wt.-%, above all 30 to 70 wt.-%, microbial oil, in particular of Stramanopiles, preferably of the class Labyrinthulea, more preferably of the genera Aurantiochytrium or Schizochytrium, and 5 to 95 wt.-%, in particular 10 to 90 wt.-% or 20 to 80 wt.-%, above all 30 to 70 wt.-%, oil of a marine organism, preferably fish oil. or vegetable oil, in particular canola oil or soy oil.

In a preferred embodiment of the invention, the added oil is a vegetable oil, in particular selected from canola oil, soy oil, rapeseed oil, palm oil, palm kernel oil, coconut oil, corn oil, olive oil, sunflower oil, cotton seed oil, linseed oil and mixtures thereof, with canola and soy oil being particularly preferred.

As the biomass of the invention is preferably a microbial biomass, in particular a biomass of the class Labyrinthulea, addition of vegetable oil preferably leads to a product which contains a mixture of a PUFAs containing microbial oil, in particular of the class Labyrinthulea, and vegetable oil. Thus, a further subject of the invention are oil compositions, comprising microbial oil, in particular of the class Labyrinthulea, preferably of the genera Aurantiochytrium and Schizochytrium, and vegetable oil, in particular canola oil, in a (w/w) ratio of 30:1 to 1:30, preferably in a (w/w) ratio of 20:1 to 1:20, more preferably in a (w/w) ratio of 10:1 to 1:10, 5:1 to 1:5 or 2:1 to 1:2.

Thus, a further subject of the invention is also a lipid, comprising 5 to 95 wt.-%, in particular 10 to 90 wt.-% or 20 to 80 wt.-%, above all 30 to 70 wt.-%, microbial oil, in particular of Stramanopiles, preferably of the class Labyrinthulea, more preferably of the genera Aurantiochytrium or Schizochytrium, and 5 to 95 wt.-%, in particular 10 to 90 wt.-% or 20 to 80 wt.-%, above all 30 to 70 wt.-%, oil of vegetable oil, in particular canola oil or soy oil.

The oil products of the invention preferably contain PUFAs in an amount of at least 10 wt.-%, preferably in an amount of at least 15, 20 or 25 wt.-%, more preferably in an amount of at least 30, 40 or 50 wt.-%.

In a preferred embodiment of the invention, in particular when a mixture of a microbial oil and a marine oil is prepared, the amount of DHA is about the same like the amount of EPA. I.e. a preferred embodiment of the invention is a lipid which contains DHA and EPA in a ratio of 2:1 to 1:2, more preferably 3:2 to 2:3, above all in a ratio of 4:3 to 3:4, wherein the lipid is preferably a mixture of microbial oil, in particular of the class Labyrinthulea, more preferably of the genera Aurantiochytrium or Schizochytrium, and marine oil, in particular fish oil.

The oil products of the invention further preferably exhibit at least one, more preferably at least 3, 5, 7 or 10, characteristic selected from: a) a peroxide value of less than 0.5, preferably less than 0.3, in particular less than 0.15; b) an anisidine value of less than 15, preferably less than 10; c) a TOTOX value of less than 25, preferably less than 20, more preferably less than 15; d) a content of free fatty acids of less than 5 wt.-%, preferably less than 3 wt.-%; e) a content of moisture and impurities of less than 1 wt.-%, preferably less than 0.5 wt.-%; f) a viscosity of less than 250 cps, more preferably of less than 200 cps, in particular of less than 160 cps; g) a flash point of at least 300°C, more preferably of at least 350°C, in particular of at least 400°C, above all of at least 450°C; h) a content of omega-3 fatty acids, in particular of DHA and EPA, of at least 35 wt.-%, preferably at least 40 or 45 wt.-%, above all at least 50 wt.-%; i) preferably DHA and EPA each in an amount of at least 8 wt.-%, preferably at least 10 wt.-%, above all at least 15 wt.-%.; j) preferably an amount of organic solvents of less than 0.5 wt.-%, more preferably less than 0.1 wt.-%, in particular less than 0.05 wt.-%, above all less than 0.01 wt.-%; k) preferably an amount of chlorides of less than 0.1 wt.-%, more preferably less than 0.05 wt.-%, in particular less than 0.01 wt.-%; I) preferably a content of crude fat of more than 90 wt.-%.

The anisidine value (AV) is determined in accordance with AOCS Official Method Cd 18-90. The AV is a measure for secondary reaction products of the fatty acids, such as aldehydes and ketones, that occur during oxidation of the oil.
The peroxide value (PV) is determined in accordance with the AOCS Official Method CD 8-53. The PV is a measure for primary reaction products, such as peroxide and hydroperoxides, that occur during oxidation of the oil. - According to the invention the PV is measured in meq/kg.

The content of free fatty acids is determined in accordance with AOCS Official Method AOCS Ca 5a-40. The content of moisture is determined in accordance with AOCS Official Methods AOAC 930.15, 935.29. The content of insoluble impurities is determined in accordance with AOCS Official Method AOCS 3a-46. The amount of DHA and EPA is determined in accordance with AOCS Official Method AOCS Ce 1b-89. The amount of total fat is determined in accordance with AOCS Official Method AOCS 996.06. The amount of crude fat is determined in accordance with AOCS Official Methods AOAC 920.39, 954.02.

In a further preferred embodiment of the invention, the oil is a PUFAs containing oil which stems from the same kind of microorganism like the oil as contained in the (demulsified) suspension.

In a very preferred embodiment of the invention, the oil which is added is oil which stems from the method as applied itself, i.e. it is oil which was produced by the method before. In other words, the oil which is obtained by mechanically separating the oil phase from the aqueous phase is recycled in part into a previous step of the isolation process to increase the oil to TDM ratio.

Thus, a further preferred subject of the invention is a method of isolating a polyunsaturated acids (PUFAs) containing lipid from a biomass, comprising the following steps:
a) Providing a demulsified PUFAs containing biomass composition;
b) Separating the oil containing light phase from the water, salt, remaining oil and cell debris containing heavy phase by mechanical means;
c) Recycling the separated oil in part to increase the oil to TDM ratio.

Thus, a particularly preferred subject of the invention is also a method of isolating a polyunsaturated fatty acids (PUFAs) containing lipid from a biomass, comprising the following steps:
a) Providing a suspension of a biomass comprising cells which contain a PUFAs containing lipid;
b) Lysing the cells of the biomass at least in part;
c) Concentrating the suspension to a total dry matter (TDM) content of 20 to 60 wt.-%, if the suspension has a lower TDM content;
d) Adjusting in the suspension a temperature of 20°C to 100°C, preferably 25, 30, 40, 50 or 60°C to 100°C, more preferably 65°C to 95°C, in particular 70 to 90°C;
e) Keeping the temperature in the range as depicted in (d) for at least 0.5 hours, preferably for at least 1, 2, 3, 4, 5, 6, 7 or 8 hours, more preferably for 1 to 36 hours, in particular for 2, 3, 4, 5, 6, 7 or 8 hours to 36 hours, more preferably 10 to 24 hours, while adding in total 7.5 to 25 moles, preferably 8.5 to 22 moles, in particular 10 to 20, more preferably 11 to 18, above all 12 to 17 moles, of base equivalent to 10 kg of total dry matter as contained in the suspension;
f) Adjusting a pH value of 5.0 to 8.5, preferably 5.0 to 7.5, more preferably 5.0 to 6.5, and incubating for 0.1 to 6 hours, preferably 0.5 to 4 hours;
g) Separating the oil containing light phase from the water, salt, remaining oil and cell debris containing heavy phase by mechanical means;
characterized in that a part of the separated oil as obtained in step (g) is added to the suspension sometime after step (b), preferably after step (e) or after step (f), more preferably after step (f), so that the ratio of oil to total dry matter (TDM) reaches a value of at least 0.5, preferably 0.5 to 0.9 or 0.5 to 0.8, in particular 0.5 to 0.7 or 0.5 to 0.65, more preferably 0.5 to 0.6.

In a particularly preferred embodiment of the invention, recycling of the oil is conducted as a continuous process, i.e. the oil which is obtained in the last step of the method after mechanical separation of the aqueous phase is recycled continuously in part into the process, preferably sometime after step (b), more preferably after step (e) or after step (f).

Alternatively, the preparation of the demulsified PUFAs containing composition is carried out by fermenting PUFAs producing cells, until the ratio of oil to TDM reaches a value of at least 0.5, preferably at least 0.5 to 0.9.

Thus, in a further preferred embodiment of the invention the preparation of the demulsified PUFAs containing composition comprises the following steps:
a) Fermenting PUFAs producing cells, until the ratio of oil to total dry matter (TDM) in the fermentation broth reaches a value of at least 0.5, preferably 0.5 to 0.9 or 0.5 to 0.8, in particular 0.5 to 0.7 or 0.5 to 0.65, more preferably 0.5 to 0.6;
b) Lysing the cells of the biomass at least in part;
c) Concentrating the suspension to a total dry matter (TDM) content of 20 to 60 wt.-%, if the suspension has a lower TDM content;
d) Adjusting in the suspension a temperature of 20°C to 100°C, preferably 25, 30, 40, 50 or 60°C to 100°C, more preferably 65°C to 95°C, in particular 70 to 90°C;
e) Keeping the temperature in the range as depicted in (d) for at least 0.5 hours, preferably for at least 1, 2, 3, 4, 5, 6, 7 or 8 hours, more preferably for 1 to 36 hours, in particular for 2, 3, 4, 5, 6, 7 or 8 hours to 36 hours, more preferably 10 to 24 hours, while adding in total 7.5 to 25 moles, preferably 8.5 to 22 moles, in particular 10 to 20, more preferably 11 to 18, above all 12 to 17 moles, of base equivalent to 10 kg of total dry matter as contained in the suspension;
f) Adjusting a pH value of 5 to 8.5, preferably 5 to 7.5, more preferably 5 to 6.5, and incubating for 0.1 to 6 hours, preferably 0.5 to 4 hours.

In the methods as disclosed before, step (e), i.e. the addition of the base equivalents, leads to the breaking of the emulsion as contained in the suspension into an oil containing light phase and a water, cell debris and salts containing heavy phase. This breaking of the emulsion is also called "demulsification" in the context of this application.

If not indicated otherwise, then the pH is according to the invention is preferably kept in the demulsification step below 11.5, more preferably below 11, above all below 10.5, in particular in the range of 7 to 11.5, more preferably in the range of 8 to 11 or 9 to 11, in particular in the range of 10 to 11. This is realized by adding the base in the demulsification step not at once, but either continuously or step by step, so that exceeding the indicated pH values can be avoided.

In a special embodiment of the invention, the demulsification is carried out at a relatively low pH of below 9.0, in particular at a pH of between 7.0 and below 9.0, preferably at a pH of between 7.0 and 8.5, in particular of between 7.5 and 8.5.

In a further special embodiment of the invention the demulsification is carried out at a temperature of below 80°C, in particular at a temperature of 20 to below 80°C, preferably at a temperature of 25 to 75°C, 30 to 75°C, 40 to 75°C, 50 to 75°C or 60 to 75°C. I.e. in particular that the temperature in the methods as disclosed before is adjusted in step (d) accordingly.

In a further special embodiment of the invention, the demulsification step is carried out for less than 12 hours, in particular for 0.5, 1, 2 or 4 to 10 hours, preferably 0.5, 1, 2 or 4 to 8 or 0.5, 1, 2 or 4 to 6 hours.

In a further special embodiment of the invention, the demulsification is carried out without prior lysing of the cells of the biomass, i.e. step (b) of the methods as mentioned above is omitted.

Preferably, for preparing the demulsified composition, the suspension is continuously mixed by using a stirrer and/or an agitator. In particular low shear agitation and/or axial-flow agitation may be applied, in particular as disclosed in WO 2015/095694. Impellers suitable for agitation include straight blade impellers, Rushton blade impellers, axial flow impellers, radial flow impellers, concave blade disc impellers, high-efficiency impellers, propellers, paddles, turbines and combinations thereof.

The term "base equivalent" takes account of the fact that not only monovalent, but also bi- or multivalent bases do exist and can be used according to the invention. In case that a bivalent base is used instead or in addition to a monovalent base, then only the half molar amount of this bivalent base has to be used to realize the same amount of base equivalents in comparison to the amount of monovalent base which would have to be used; in case a trivalent base is used, then only a third of the molar amount of monovalent base has to be used; etc. In case that a monovalent base, like sodium hydroxide, is used, the amount of base equivalents is identical to the amount of base.

The weight amount of base can easily be calculated basing on the molar amount by making use of the molar weight of the base. For example, in case of the very preferred base sodium hydroxide the molar weight equals to 40 g/mol. That means that one mole of sodium hydroxide corresponds to 40 g of sodium hydroxide.

Preferred bases as used according to the invention are selected from hydroxides, in particular sodium hydroxide, lithium hydroxide, potassium hydroxide, and/or calcium hydroxide, carbonates, in particular sodium carbonate, potassium carbonate, and/or magnesium carbonate, and/or bicarbonates, in particular lithium bicarbonate, sodium bicarbonate, and/or potassium bicarbonate. In a very preferred embodiment of the invention, the base used according to the invention is only or almost only sodium hydroxide. - Due to easiness of handling, the bases are preferably used in liquid form, in particular as concentrated solutions, wherein the concentration of base in the solution is preferably in the range of 10 to 60 wt.-%, in particular in the range of 20 to 50 wt.-%.

According to the invention after providing the biomass containing suspension preferably a lysing step is carried out. The lysing step can be omitted, if - for example due to the fermentation conditions as applied - the cells or a big part thereof is already lysed or easily breakable in one of the following steps of the procedure without any explicit lysing step.

Lysing of the cells of the biomass can be carried out by methods as known to those skilled in the art, in particular enzymatically, mechanically, physically, or chemically, or by applying combinations thereof.

Depending on the time of exposure and/or the degree of force applied, a composition comprising only lysed cells or a composition comprising a mixture of cell debris and intact cells may be obtained. The term "lysed lipids containing biomass" insofar relates to a suspension which contains water, cell debris and oil as set free by the cells of the biomass, but beyond that may also comprise further components, in particular salts, intact cells, further contents of the lysed cells as well as components of a fermentation medium, in particular nutrients. According to the invention, "lysing the cells at least in part" means that at least 20%, preferably at least 40, 60 or 80% of the cells as contained in the suspension are lysed.

In a preferred embodiment of the invention, only small amounts of intact cells, in particular less than 20 %, preferably less than 10 %, more preferably less than 5 %, of the cells (relating to the total number of intact cells as present before lysing the cells of the biomass) are present in the lysed biomass or biomass suspension after the step of lysing the cells has been carried out.

Lysing of the cells may be realized for example by utilizing a French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer.

In a preferred embodiment of the invention, lysing of the cells comprises an enzymatic treatment of the cells by applying a cell-wall degrading enzyme.

According to the invention, the cell-wall degrading enzyme is preferably selected from proteases, cellulases (e.g., Cellustar CL (Dyadic), Fibrezyme G2000 (Dyadic), Celluclast (Novozymes), Fungamyl (Novozymes), Viscozyme L (Novozymes)), hemicellulases, chitinases, pectinases (e.g., Pectinex (Novozymes)), sucrases, maltases, lactases, alpha-glucosidases, beta-glucosidases, amylases (e.g., Alphastar Plus (Dyadic); Termamyl (Novozymes)), lysozymes, neuraminidases, galactosidases, alpha-mannosidases, glucuronidases, hyaluronidases, pullulanases, glucocerebrosidases, galactosylceramidases, acetylgalactosaminidases, fucosidases, hexosaminidases, iduronidases, maltases-glucoamylases, xylanases (e.g., Xylanase Plus (Dyadic), Pentopan (Novozymes)), beta-glucanases (e.g., Vinoflow Max (Novozymes), Brewzyme LP (Dyadic)), mannanases, and combinations thereof. The protease may be selected from serine proteases, threonine proteases, cysteine proteases, aspartate proteases, metalloproteases, glutamic acid proteases, alcalases (subtilisins), and combinations thereof. The chitinase may be a chitotriosidase. The pectinase may be selected from pectolyases, pectozymes, polygalacturonases, and combinations thereof.

The adequate pH for utilizing the enzyme depends on the pH optimum of the enzyme.

In a preferred embodiment of the invention, an enzyme with a pH optimum of between 7.0 and 8.0, in particular of about 7.5, is used, so that the pH applied in this step is from 7.0 to 8.0, preferably from 7.3 to 7.7. A preferred enzyme which can be used in this pH range is an alcalase.

The enzyme is preferably added as a concentrated enzyme solution, in particular in an amount of 0.01 to 1.5 wt.-%, preferably in an amount of 0.03 to 1.0 wt.-%, more preferably in an amount of 0.05 to 0.5 wt.-%, relating to the amount of concentrated enzyme solution as added in relation to the total amount of the suspension after addition of the concentrated enzyme solution.

In a very preferred embodiment of the invention, lysing of the cells is carried out as follows:
i) Heating the biomass suspension to a temperature of between 50°C and 70°C, preferably to a temperature of between 55°C and 65°C, and adding a cell wall-degrading enzyme to the suspension, in particular fermentation broth, and adjusting an adequate pH value, if necessary, at which the enzyme is properly working;
ii) Keeping the temperature and pH in the ranges as depicted in (ii) for at least one hour, preferably for at least two hours, more preferably for two to four hours.

In step (i), the enzyme can be added before or after heating up the suspension and/or before or after adjusting the pH. In the same way heating up of the suspension can be carried out before or after adjusting the pH. - But in a preferred embodiment, the enzyme is added after heating up of the suspension and after adjusting the pH, if adjusting of the pH is necessary, at all. - In a very preferred embodiment all measures are carried out more or less simultaneously.

Preferably, in the steps (i) and (ii) the suspension is continuously mixed by using a stirrer and/or an agitator.

According to the invention, the demulsification is preferably carried out with a suspension having a dry matter content of 20 to 60 wt.-%, more preferably 25 to 60 wt.%, in particular 30 to 55 wt.-% or 30 to 45 wt.-%. This can be realized by either providing a suspension with an appropriately high biomass or by concentrating the biomass suspension, in particular after lysing the cells of the biomass. Thus, in a preferred embodiment of the invention, after optional lysing of the cells of the biomass and before the demulsification step, the suspension is concentrated to a total dry matter content of 20 to 60 wt.-%, more preferably 25 to 60 wt.-%, in particular 30 to 55 wt.-%, above all 30 to 50 wt.-% or 30 to 45 wt.-%.

Concentration of the suspension is preferably carried out by evaporation of water at a temperature not higher than 100°C, preferably 70°C to 100°C, more preferably 80°C to 90°C, until a total dry matter content of 20 to 60 wt.-% more preferably 25 to 60 wt.-%, in particular 30 to 55 wt.-% or 30 to 45 wt.-%, is reached.

Concentration of the suspension is preferably carried out in a forced circulation evaporator (for example available from GEA, Germany) to allow fast removal of the water. Alternatively or in addition, concentration might be carried out by falling film evaporation, thin film evaporation and/or rotary evaporation.

In general, adjusting the pH value can be carried out according to the invention by using either bases or acids as known to those skilled in the art. Decreasing of the pH can be carried out in particular by using organic or inorganic acids like sulfuric acid, nitric acid, phosphoric acid, boric acid, hydrochloric acid, hydrobromic acid, perchloric acid, hypochlorous acid, chlorous acid, fluorosulfuric acid, hexafluorophosphoric acid, acetic acid, citric acid, formic acid, or combinations thereof. As a high content of chloride is desirably avoided, in a preferred embodiment of the invention no or only small amounts of hydrochloric acid are used in the process of the invention. According to the invention, sulfuric acid is the preferred substance for decreasing the pH value. - Increasing of the pH can be carried out in particular by using organic or inorganic bases like hydroxides, in particular sodium hydroxide, lithium hydroxide, potassium hydroxide, and/or calcium hydroxide, carbonates, in particular sodium carbonate, potassium carbonate, or magnesium carbonate, and/or bicarbonates, in particular lithium bicarbonate, sodium bicarbonate, and/or potassium bicarbonate. - Due to easiness of handling, the acids and bases are preferably used in liquid form, in particular as concentrated solutions, wherein the concentration of acid or base in the solution is preferably in the range of 10 to 55 wt.-%, in particular in the range of 20 to 50 wt.-%. In particular sulfuric acid is preferably used also in concentrated form.

The method according to the invention comprises as a further step the harvesting of the PUFAs containing lipid from the demulsified composition.

The harvesting of the PUFAs containing lipid preferably comprises "neutralization" of the demulsified suspension and subsequent separation of the thus obtained oil containing light phase from the water, salts, cell debris and residual oil containing heavy phase.

"Neutralization" of the demulsified composition (step (f) of the methods of the invention) is preferably realized by adding an acid, preferably sulfuric acid, to adjust a pH value of 5.0 to 8.5, in particular 5.0 to 7.5, preferably 5.0 to 7.0. In a preferred embodiment of the invention a pH below 7, preferably a pH of 5.0 to 6.5, 5.5 to 6.5 or 5.0 to 6.0, is adjusted, as higher oil yields were observed at pH values below 7.

Before starting separation of the light phase from the heavy phase the neutralized composition may be stirred at this neutralized pH from several minutes up to several hours, preferably from 0.1 to 6 hours, more preferably from 0.5 to 4 hours.

"Neutralization" of the demulsified composition as described before is only necessary, if the demulsified composition as obtained according to step (e) of the methods of the invention has a pH value outside of this range.

Separation of the oil containing light phase from the water, salts and cell debris containing heavy phase is preferably realized by mechanical means and preferably at a temperature of 60-90°C, more preferably 70-80°C, and preferably at a pH value of 6-9, more preferably 7-8.5. According to the invention, "mechanical means" refers in particular to filtration and centrifugation methods as known to those skilled in the art. In a preferred embodiment of the invention, separation of the oil containing light phase from the aqueous phase is carried out by a two step centrifugation process, in particular as disclosed in WO 2019/032880.

After separation of the oil containing light phase, the PUFAs containing oil thus obtained can further be worked up by applying methods as known to those skilled in the art, in particular refining, bleaching, deodorizing and/or winterizing.

A particular advantage of the method of the invention is that it can be carried out without the use of any organic solvent, in particular without the use of any polar or non-polar organic solvent. Thus, in a preferred embodiment of the invention, no or only little amounts of organic solvents, in particular of polar or non-polar organic solvents, are used for isolating the PUFAs containing oil from the biomass. Typical organic solvents are hexane and ethanol.

In a preferred embodiment of the invention less than 2 wt.-% non-polar organic solvents are used, more preferably less than 1, 0.5 or 0.1 wt.-%. In a particularly preferred embodiment of the invention no non-polar organic solvent is used, at all. In a very preferred embodiment of the invention less than 2 wt.-% organic solvents are used, in general, particularly preferred less than 1, 0.5 or 0.1 wt.-%. In a particularly very preferred embodiment of the invention no organic solvents are used, at all.

A further advantage of the method of the invention is that a very efficient separation of the oil from the remaining biomass can be realized without the addition of sodium chloride, which is normally used for salting out the oil from the biomass. Preferably the method can be carried out without the addition of chloride salts, at all, above all without the addition of any salts for salting out the oil. But small amounts of chloride salts, in particular sodium chloride, might be present in the suspension due to the fermentation medium as used for growing of the biomass.

Thus, in a preferred embodiment of the invention, no or only little amounts of sodium chloride are used for improving the oil isolation. In a preferred embodiment of the invention less than 1 wt.-% of sodium chloride, are used, more preferably less than 0.5 or 0.2 wt.-% of sodium chloride are used for isolating the oil from the biomass, above all less than 0.1 or 0.05 wt.-%, wherein the wt.-% relate to the total weight of the composition after addition of the sodium chloride.

In a particularly preferred embodiment of the invention no or only little amounts of chloride salts are used for improving the oil isolation, at all. In this embodiment preferably less than 1 wt.-% of chloride salts, more preferably less than 0.5 or 0.2 wt.-% of chloride salts are used for isolating the oil from the biomass, above all less than 0.1 or 0.05 wt.-%, wherein the wt.-% relate to the total weight of the composition after addition of the chloride salts.

In a very preferred embodiment of the invention no or only little amounts of salts are used for improving the oil isolation, in general. In this embodiment preferably less than 1 wt.-% of salts, more preferably less than 0.5 or 0.2 wt.-% of salts are used for isolating the oil from the biomass, above all less than 0.1 or 0.05 wt.-%, wherein the wt.-% relate to the total weight of the composition after addition of the salts.

The methods of the invention allow a very efficient separation of the oil contained in the biomass from the cell-debris and other substances as contained in the suspension, in particular fermentation broth. By using the methods of the invention preferably more than 90 wt.-%, in particular more than 95, 98 or 99 wt.-% of the oil contained in the biomass can be separated from the biomass and isolated by applying very economic and sustainable conditions.

"Chloride" according to the invention refers to the amount of detectable chlorine. The amount of chlorine as present can be determined for example by elemental analysis according to DIN EN ISO 11885. The chlorine is present in the form of salts which are called "chlorides". The content of chloride as mentioned according to the invention - also called "chloride ions" - only refers to the amount of detectable chlorine, not to the amount of the complete chloride salt, which comprises besides the chloride ion also a cationic counterion.

The total dry matter (TDM), in particular of the suspension, relates to the residual weight of the suspension after drying has been carried out, i.e. after water has been removed. Thus, the TDM does not only relate to cell debris and salts, but in particular also to the oil as contained in the suspension.

The TDM content is preferably determined by gravimetric analysis. For doing that, a homogeneous sample with a specific volume is weighed before and after freeze-drying. The remaining weight of the dried sample corresponds to the total dry matter as contained in that specific volume. The TDM content of the sample is the quotient between the mass of the sample after freeze-drying and the mass of the sample before freeze-drying.

The amount of oil in the sample is preferably determined by fatty acid methyl ester analysis (FAME). For doing that the lipids in the sample are first saponified with KOH. After that, the free fatty acids are methylated with MeOH. The methylated fatty acids can then be determined and quantified by gas chromatography by using an internal standard.

In a special embodiment of the invention, the water, salts, residual oil and cell debris containing aqueous phase, which is obtained as by-product in the oil harvesting step as described before, is converted into a dried biomass by drying the biomass to a total dry matter content of more than 90 wt.-%.

Conversion of the water, salts, remaining oil and cell debris containing heavy phase, which is obtained as by-product in the oil harvesting step, into a dried biomass by drying the biomass to a total dry matter content of more than 90 wt.-%, can be carried out in different ways.

In a very preferred way, the transformation is carried out by concentration of the heavy phase to a dry matter content of 30-50 wt.-%, preferably 35-45 wt.-%, and subsequent spray granulation of the biomass by means of fluidized bed granulation. By doing that, in a very efficient way, a biomass with advantageous features can be obtained. Spray granulation by means of fluidized bed granulation is disclosed in more detail in EP13176661.0.

Concentration of the heavy phase to a dry matter content of 30-50 wt.-% is preferably carried out by solvent evaporation, in particular vacuum evaporation, and/or by using a rotary evaporator, a thin-film evaporator or a falling-film evaporator. A useful alternative to solvent evaporation is reverse osmosis.

As alternative to the spray-granulation other drying methods, in particular other convective drying methods, like tunnel drying or spray drying, in particular nozzle spray drying, or contact drying methods, like drum drying, or radiation drying methods, like infrared drying, of the concentrated heavy phase would be applicable alternatives, wherein by using those methods normally particles with a smaller or bigger diameter are obtained.

According to the invention, during the drying process, an anti-caking agent, in particular silica, preferably a hydrophobic or hydrophilic silica, may optionally be added to the biomass to prevent caking. For this purpose, the suspension, in particular fermentation broth, comprising biomass as well as the silica are preferably sprayed into the particular drying zone. Alternatively or additionally, the biomass may be mixed with the anti-caking agent after the drying process. With respect to the use of silica as anti-caking agent reference is made in particular to the patent application EP13187631.0.

Conversion of a fine-grained powder into a coarse-grained dust-free product can be realized by granulating processes. Conventional organic or inorganic auxiliaries or supports such as starch, gelatin, cellulose derivatives or similar substances, which are typically used in food processing or feed processing as binding agents, gelling agents or thickeners, may optionally be used in this subsequent granulation process. Further auxiliaries that are preferably used according to the invention are disclosed in WO 2016/050560, with carboxymethylcellulose being a particulary preferred binding agent.
After drying and optionally granulating and/or sieving of the biomass, the dried biomass is preferably stored or packed.

The particulate biomass of the invention as well as the aqueous suspensions of the invention can be used in different ways. For example, they can be used in order to produce a foodstuff or feedstuff. Alternatively they may be used directly as foodstuff or feedstuff.

A further subject matter of the present invention is therefore likewise a method for producing a feedstuff or foodstuff, in which a particulate biomass and/or an aqueous suspension according to the invention is used, and is preferably mixed with further feedstuff or foodstuff ingredients.

The PUFAs containing cells of the biomass are preferably microbial cells or plant cells. Preferably, the cells are capable of producing the PUFAs due to a polyketide synthase system. The polyketide synthase system may be an endogenous one or, due to genetic engineering, an exogenous one.

The plant cells may in particular be selected from cells of the families Brassicaceae, Elaeagnaceae and Fabaceae. The cells of the family Brassicaceae may be selected from the genus Brassica, in particular from oilseed rape, turnip rape and Indian mustard; the cells of the family Elaeagnaceae may be selected from the genus Elaeagnus, in particular from the species Oleae europaea; the cells of the family Fabaceae may be selected from the genus Glycine, in particular from the species Glycine max.

The microbial organisms which contain a PUFAs containing lipid are described extensively in the prior art. The cells used may, in this context, in particular be cells which already naturally produce PUFAs (polyunsaturated fatty acids); however, they may also be cells which, as the result of suitable genetic engineering methods or due to random mutagenesis, show an improved production of PUFAs or have been made capable of producing PUFAs, at all. The production of the PUFAs may be auxotrophic, mixotrophic or heterotrophic.

The biomass preferably comprises cells which produce PUFAs heterotrophically. The cells according to the invention are preferably selected from algae, fungi, particularly yeasts, bacteria, or protists. The cells are more preferably microbial algae or fungi.

Suitable cells of oil-producing yeasts are, in particular, strains of Yarrowia, Candida, Rhodotorula, Rhodosporidium, Cryptococcus, Trichosporon and Lipomyces.

Suitable cells of oil-producing microalgae and algae-like microorganisms are, in particular, microorganisms selected from the phylum Stramenopiles (also called Heterokonta). The microorganisms of the phylum Stramenopiles may in particular be selected from the following groups of microorganisms: Hamatores, Proteromonads, Opalines, Developayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinales, Pedinellales, Dictyochales), Chrysomeridales, Sarcinochrysidales, Hydrurales, Hibberdiales, and Chromulinales. Other preferred groups of microalgae include the members of the green algae and dinoflagellates, including members of the genus Crypthecodiurn.

The biomass according to the invention preferably comprises cells, and preferably consists essentially of such cells, of the taxon Labyrinthulomycetes (Labyrinthulea, net slime fungi, slime nets), in particular those from the family of Thraustochytriaceae. The family of the Thraustochytriaceae (Thraustochytrids) includes the genera Althornia, Aplanochytrium, Aurantiochytrium, Botryochytrium, Elina, Japonochytrium, Oblongichytrium, Parietichytrium, Schizochytrium, Sicyoidochytrium, Thraustochytrium, and Ulkenia. The biomass particularly preferably comprises cells from the genera Aurantiochytrium, Oblongichytrium, Schizochytrium, or Thraustochytrium, above all from the genus Schizochytrium.

In accordance with the invention, the polyunsaturated fatty acid (PUFA) is preferably a highly-unsaturated fatty acid (HUFA).

The cells present in the biomass are preferably distinguished by the fact that they contain at least 20% by weight, preferably at least 30% by weight, in particular at least 35% by weight, of PUFAs, in each case based on cell dry matter.

According to the invention, the term "lipid" includes phospholipids; free fatty acids; esters of fatty acids; triacylglycerols; sterols and sterol esters; carotenoids; xanthophylls (e. g. oxycarotenoids); hydrocarbons; isoprenoid-derived compounds and other lipids known to one of ordinary skill in the art. - The terms "lipid" and "oil" are used interchangeably according to the invention.

In a preferred embodiment, the majority of the lipids in this case is present in the form of triglycerides, with preferably at least 50% by weight, in particular at least 75% by weight and, in an especially preferred embodiment, at least 90% by weight of the lipids present in the cell being present in the form of triglycerides.

According to the invention, polyunsaturated fatty acids (PUFAs) are understood to mean fatty acids having at least two, particularly at least three, C-C double bonds. According to the invention, highly-unsaturated fatty acids (HUFAs) are preferred among the PUFAs. According to the invention, HUFAs are understood to mean fatty acids having at least four C-C double bonds.

The PUFAs may be present in the cell in free form or in bound form. Examples of the presence in bound form are phospholipids and esters of the PUFAs, in particular monoacyl-, diacyl- and triacylglycerides. In a preferred embodiment, the majority of the PUFAs is present in the form of triglycerides, with preferably at least 50% by weight, in particular at least 75% by weight and, in an especially preferred embodiment, at least 90% by weight of the PUFAs present in the cell being present in the form of triglycerides.

Preferred PUFAs are omega-3 fatty acids and omega-6 fatty acids, with omega-3 fatty acids being especially preferred. Preferred omega-3 fatty acids are eicosapentaenoic acid (EPA, 20:5ω-3), particularly (5*Z*,8*Z*,11*Z*,14*Z*,17*Z*)-eicosa-5,8,11,14,17-pentaenoic acid, and docosahexaenoic acid (DHA, 22:6ω-3), particularly (4*Z*,7*Z*,10*Z*,13*Z*,16*Z*,19*Z*)-docosa-4,7,10,13,16,19-hexaenoic acid.

In a very preferred embodiment of the invention, cells, in particular a Schizochytrium strain, is employed which produces a significant amount of EPA and DHA, simultaneously, wherein DHA is preferably produced in an amount of at least 20 wt.-%, preferably in an amount of at least 30 wt.-%, in particular in an amount of 30 to 50 wt.-%, and EPA is produced in an amount of at least 5 wt.-%, preferably in an amount of at least 10 wt.-%, in particular in an amount of 10 to 20 wt.-% (in relation to the total amount of lipid as contained in the cells, respectively). DHA and EPA producing *Schizochytrium* strains can be obtained by consecutive mutagenesis followed by suitable selection of mutant strains which demonstrate superior EPA and DHA production and a specific EPA:DHA ratio. Any chemical or nonchemical (e.g. ultraviolet (UV) radiation) agent capable of inducing genetic change to the yeast cell can be used as the mutagen. These agents can be used alone or in combination with one another, and the chemical agents can be used neat or with a solvent.

Preferred species of microorganisms of the genus Schizochytrium, which produce EPA and DHA simultaneously in significant amounts, as mentioned before, are deposited under ATCC Accession No. PTA-10208, PTA-10209, PTA-10210, or PTA-10211, PTA-10212, PTA-10213, PTA-10214, PTA-10215.

The suspension of biomass according to the present invention has preferably a biomass density of at least 80 or 100 g/l, in particular a biomass density of 80 to 250 g/l, in particular 80 to 200 g/l, more preferably a biomass density of at least 120 or 140 g/l, in particular 120 or 140 to 250 g/l, above all a biomass density of at least 160 or 180 g/l (calculated as dry-matter content) and is preferably a fermentation broth. Thus, the suspension may be obtained by culturing and growing suitable cells in a fermentation medium under conditions whereby the PUFAs are produced by the microorganism.

Methods for producing the biomass, in particular a biomass which comprises cells containing lipids, in particular PUFAs, particularly of the order Thraustochytriales, are described in detail in the prior art (see e.g. WO91/07498, WO94/08467, WO97/37032, WO97/36996, WO01/54510). As a rule, the production takes place by cells being cultured in a fermenter in the presence of a carbon source and of a nitrogen source, along with a number of additional substances like minerals that allow growth of the microorganisms and production of the PUFAs. In this context, biomass densities of more than 100 grams per litre and production rates of more than 0.5 gram of lipid per litre per hour may be attained. The process is preferably carried out in what is known as a fed-batch process, i.e. the carbon and nitrogen sources are fed in incrementally during the fermentation. When the desired biomass has been obtained, lipid production may be induced by various measures, for example by limiting the nitrogen source, the carbon source or the oxygen content or combinations of these.

In a preferred embodiment of the invention, the cells are grown until they reach a biomass density of at least 80 or 100 g/l, more preferably at least 120 or 140 g/l, in particular at least 160 or 180 g/l (calculated as total dry matter content). Such processes are for example disclosed in US 7,732,170.

Preferably, the cells are fermented in a medium with low salinity, in particular so as to avoid corrosion. This can be achieved by using chlorine-free sodium salts as the sodium source instead of sodium chloride, such as, for example, sodium sulphate, sodium carbonate, sodium hydrogen carbonate or soda ash. Preferably, chloride is used in the fermentation in amounts of less than 3 g/l, in particular less than 500 mg/l, especially preferably less than 100 mg/l.

Suitable carbon sources are both alcoholic and non-alcoholic carbon sources. Examples of alcoholic carbon sources are methanol, ethanol and isopropanol. Examples of non-alcoholic carbon sources are fructose, glucose, sucrose, molasses, starch and corn syrup.

Suitable nitrogen sources are both inorganic and organic nitrogen sources. Examples of inorganic nitrogen sources are nitrates and ammonium salts, in particular ammonium sulphate and ammonium hydroxide. Examples of organic nitrogen sources are amino acids, in particular glutamate, and urea.

In addition, inorganic or organic phosphorus compounds and/or known growth-stimulating substances such as, for example, yeast extract or corn steep liquor, may also be added so as to have a positive effect on the fermentation.

The cells are preferably fermented at a pH of 3 to 11, in particular 4 to 10, and preferably at a temperature of at least 20°C, in particular 20 to 40°C, especially preferably at least 30°C. A typical fermentation process takes up to approximately 100 hours.

After the fermentation has ended, the cells may be pasteurized in order to kill the cells and to deactivate enzymes which might promote lipid degradation. The pasteurization is preferably affected by heating the biomass to a temperature of 50 to 121°C, preferably 50 to 70°C, for a period of 5 to 150 minutes, in particular 20 to 100 minutes.

Likewise, after the fermentation is ended, antioxidants may be added in order to protect the PUFAs present in the biomass from oxidative degradation. Preferred antioxidants in this context are BHT, BHA, TBHA, ethoxyquin, beta-carotene, vitamin E, in particular tocopherol, and vitamin C. The antioxidant, if used, is preferably added in an amount of 0.001 to 0.1 wt.-%, preferably in an amount of 0.002 to 0.05 wt.-%, relating to the total amount of the fermentation broth after addition of the antioxidant.

### Working examples

### Example 1: Preparation of the suspension for use in the demulsification tests

An unwashed cell broth containing microbial cells (Schizochytrium sp.) at a biomass density of over 100 g/l was heated to 60°C in an agitated vessel. After heating up the suspension, the pH was adjusted to 7.5 by using caustic soda (50 wt.-% NaOH solution), before an alcalase (Alcalase^{®} 2.4 FG (Novozymes)) was added in liquid form in an amount of 0.5 wt.-% (by weight broth). Stirring was continued for 3 hours at 60°C. After that, the lysed cell mixture was transferred into a rotary evaporator and heated to a temperature of 80°C at a reduced pressure of 400 mbar. The mixture was concentrated in the rotary evaporator, until a total dry matter content of about 35 wt.-% was reached.

As the next step, demulsification of the concentrated suspension was carried out. All experiments were carried out with about one liter of enzymatically treated and subsequently concentrated fermentation broth using a stirring vessel BIOSTAT^{®} B-DCU-Quad 2L (Sartorius, Germany). Demulsification was carried out for 24 hours at a temperature of 90°C. The suspension was stirred with 300 rpm. 1.5 mol of caustic soda per kg TDM was added continually over a period of 3 hours. After 24 hours, the demulsified compositions were neutralized to either pH 5.5 or pH 7.5. After neutralization, the TDM content and oil content were determined to find out the ratio of oil to TDM. Subsequently different amounts of oil were added to investigate the influence of the oil to TDM ratio on the final oil yield. As oil either Schizochytrium oil as obtained by the method of the invention before or vegetable oil (canola oil or soya oil) were added. About two hours after the optional addition of oil, a 50 g sample of the homogenized suspension was taken and separation of the cell debris was carried out by centrifugation at 13.500 g. Subsequently the amount of EPA and DHA in the supernatant was determined.

**Table 1: Influence of the TDM to oil ratio on the oil yield**

| Initial mass [g] | Initial oil [g] | Added oil [g] | Total oil [g] | Oil to TDM ratio | Recovery of oil at pH 7.5 | Recovery of oil at pH 5.5 | Yield at pH 7.5 | Yield at pH 5.5 |
|---|---|---|---|---|---|---|---|---|
| 1004 | 205.1 | 0 | 205.1 | 0.54 | 193.0 | 201.0 | 94.1% | 98.0% |
| 1000 | 204.3 | 21.5 | 225.8 | 0.57 | 214.8 | 221.1 | 94.6% | 97.7% |
| 1009 | 206.1 | 48.5 | 254.6 | 0.61 | 243.8 | 250.7 | 94.8% | 98.1% |
| 1002 | 204.7 | 119 | 323.7 | 0.70 | 313.3 | 319.8 | 94.9% | 98.1% |
| 1007 | 205.7 | 0 | 205.7 | 0.44 | 192.1 | 198.0 | 93.4% | 96.2% |
| 1011 | 206.5 | 30.1 | 236.6 | 0.49 | 223.9 | 228.8 | 93.8% | 96.2% |
| 1008 | 205.9 | 0 | 205.9 | 0.44 | 193.6 | 197.8 | 94.0% | 96.0% |
| 1012 | 206.7 | 107.2 | 313.9 | 0.60 | 302.3 | 308.5 | 94.4% | 97.4% |

The experimental data show that when the ratio of oil to TDM exceeds the value of 0.5, the overall oil yield is increased significantly. It does not play a role here, whether this value is reached by fermenting the cells, until the ratio of oil to TDM exceeds this value or by recycling Schizochytrium oil during the demulsification, before the separation of the oil containing light phase from the aqueous phase takes place. Further it can be seen that when after the demulsification a pH value of 5.5 is adjusted, the oil yield is much higher in comparison to adjusting a pH value of 7.5.

**Table 2: Influence of the TDM to oil ratio on the oil yield; addition of canola oil**

| Initial mass [g] | Initial oil [g] | Added oil [g] | Total oil [g] | Oil to TDM ratio | Recovery of oil at pH 7.5 | Yield at pH 7.5 |
|---|---|---|---|---|---|---|
| 1008 | 134.1 | 0 | 134.1 | 0.42 | 125.7 | 93.7% |
| 1008 | 134.1 | 171.2 | 305.3 | 0.60 | 290.4 | 95.1% |

The experimental data show that the effect which was reached with recycling Schizochytrium oil can also be realized by adding vegetable oils, in particular canola oil.

**Table 3: Influence of the TDM to oil ratio on the oil yield; addition of soy oil**

| Initial mass [g] | Initial oil [g] | Added oil [g] | Total oil [g] | Oil to TDM ratio | Recovery of oil at pH 7.5 | Yield at pH 7.5 |
|---|---|---|---|---|---|---|
| 1004 | 133.9 | 0 | 133.9 | 0.42 | 125.3 | 93.6% |
| 1000 | 133.9 | 173.2 | 305.3 | 0.60 | 291.7 | 95.0% |

The experimental data show that the effect which was reached with recycling Schizochytrium oil can also be realized by adding soy oil.

## Claims

1. A method of isolating a polyunsaturated fatty acids (PUFAs) containing lipid from a biomass, comprising the following steps:
a) Providing a demulsified PUFAs containing composition, **characterized in that** the ratio of oil to total dry matter (TDM) in the suspension is at least 0.5, preferably 0.5 to 0.9, more preferably 0.5 to 0.65;
b) Separating the oil containing light phase from the water, salt, remaining oil and cell debris containing heavy phase by mechanical means.

2. The method according to claim 1, **characterized in that** the suspension according to (a) has a TDM content of 20 to 60 wt.-%, preferably 25 to 55 wt.-%, in particular 30 to 50 wt.-%.

3. The method according to any of the preceding claims, **characterized in that** the preparation of the demulsified PUFAs containing composition comprises the following steps:
a) Providing a suspension of a biomass comprising cells which contain a PUFAs containing lipid;
b) Lysing the cells of the biomass at least in part;
c) Concentrating the suspension to a total dry matter (TDM) content of 20 to 60 wt.-%, if the suspension has a lower TDM content;
d) Adjusting in the suspension a temperature of 20°C to 100°C, preferably 25, 30, 40, 50 or 60°C to 100°C, more preferably 65°C to 95°C, in particular 70 to 90°C;
e) Keeping the temperature in the range as depicted in (d) for at least 0.5 hours, preferably for at least 1, 2, 3, 4, 5, 6, 7 or 8 hours, more preferably for 1 to 36 hours, in particular for 2, 3, 4, 5, 6, 7 or 8 hours to 36 hours, more preferably 10 to 24 hours, while adding in total 7.5 to 25 moles, preferably 8.5 to 22 moles, in particular 10 to 20, more preferably 11 to 18, above all 12 to 17 moles, of base equivalent to 10 kg of total dry matter as contained in the suspension;
f) Adjusting a pH value of 5.0 to 8.5, preferably 5.0 to 7.5, more preferably 5.0 to 6.5, and incubating for 0.1 to 6 hours, preferably 0.5 to 4 hours;
**characterized in that** oil is added to the suspension sometime after step (b), preferably after step (e) or after step (f), more preferably after step (f), so that the ratio of oil to total dry matter (TDM) reaches a value of at least 0.5, preferably 0.5 to 0.9 or 0.5 to 0.8, in particular 0.5 to 0.7 or 0.5 to 0.65, more preferably 0.5 to 0.6.

4. The method according to claim 3, wherein the oil which is added to the suspension is a microbial oil, in particular oil of Stramanopiles, an oil of marine organisms, in particular fish oil, or a vegetable oil, in particular canola oil or soya oil, or a mixture thereof.

5. The method according to any of claims 1 or 2, **characterized in that** the preparation of the demulsified PUFAs containing composition comprises the following steps:
a) Fermenting PUFAs producing cells, until the ratio of oil to total dry matter (TDM) in the fermentation broth reaches a value of at least 0.5, preferably 0.5 to 0.9 or 0.5 to 0.8, in particular 0.5 to 0.7 or 0.5 to 0.65, more preferably 0.5 to 0.6.;
b) Lysing the cells of the biomass at least in part;
c) Concentrating the suspension to a total dry matter (TDM) content of 20 to 60 wt.-%, if the suspension has a lower TDM content;
d) Adjusting in the suspension a temperature of 20°C to 100°C, preferably 25, 30, 40, 50 or 60°C to 100°C, more preferably 65°C to 95°C, in particular 70 to 90°C;
e) Keeping the temperature in the range as depicted in (d) for at least 0.5 hours, preferably for at least 1, 2, 3, 4, 5, 6, 7 or 8 hours, more preferably for 1 to 36 hours, in particular for 2, 3, 4, 5, 6, 7 or 8 hours to 36 hours, more preferably 10 to 24 hours, while adding in total 7.5 to 25 moles, preferably 8.5 to 22 moles, in particular 10 to 20, more preferably 11 to 18, above all 12 to 17 moles, of base equivalent to 10 kg of total dry matter as contained in the suspension;
f) Adjusting a pH value of 5.0 to 8.5, preferably 5.0 to 7.5, more preferably 5.0 to 6.5, and incubating for 0.1 to 6 hours, preferably 0.5 to 4 hours.

6. The method according to claim 4 or 5, wherein the pH is kept in step (e) below 11.5, preferably below 11, more preferably below 10.5, in particular in the range of 6.0 to 11.5, preferably in the range of 6.0 to 10.5, or in the range of 7.0 to 11.5, preferably in the range of 7.0 to 10.5, or in the range of 8.0 to 11.5, preferably in the range of 8.0 to 10.5, or in the range of 9.0 to 11.5, preferably in the range of 9.0 to 10.5.

7. The method according to any of the preceding claims, wherein the base is selected from hydroxides, in particular sodium hydroxide, lithium hydroxide, potassium hydroxide, and/or calcium hydroxide, and/or carbonates, in particular sodium carbonate, potassium carbonate, and/or magnesium carbonate, and/or bicarbonates, in particular lithium bicarbonate, sodium bicarbonate, and/or potassium bicarbonate, wherein sodium hydroxide is preferred.

8. The method according to any of claims 3 to 7, wherein the suspension as provided in step (a) has already a total dry matter content of 20 to 60 wt.-%, preferably 25 to 60 wt.-%, more preferably 30 to 55 wt.-%.

9. The method according to any of the preceding claims, wherein for lysing the cells no or only small amounts of salts are used, wherein "small amounts" preferably means that salts are added in an amount of less than 0.1 g/l fermentation broth.

10. The method according to any of the preceding claims, wherein for lysing the cells no or only small amounts of organic solvents are used, wherein "small amounts" preferably means that organic solvents are added in an amount of less than 0.1 g/l fermentation broth.

11. The method according to any of the preceding claims, wherein the suspension is provided as a fermentation broth with a biomass density of preferably at least 80, 100, 120 or 140 g/l, more preferably of 80 to 250, 100 to 250, 120 to 250 or 140 to 250 g/l.

12. The method according to any of the preceding claims, wherein the cells which contain a PUFAs containing lipid are selected from algae, fungi, protists, bacteria, microalgae, plant cells, and mixtures thereof, wherein the microalgae are preferably selected from the phylus Stramanopiles, in particular of the family of Thraustochytrids, preferably of the genus Schizochytrium.

13. Lipid comprising a mixture of a microbial oil, in particular of Stramanopiles, preferably of the class Labyrinthulea, more preferably of the genera Aurantiochytrium or Schizochytrium, and at least one oil selected from oils of marine organisms, preferably fish oil, and vegetable oils, preferably canola oil or soy oil.

14. Lipid according to claim 13, comprising 5 to 95 wt.-%, in particular 10 to 90 wt.-% or 20 to 80 wt.-%, above all 30 to 70 wt.-%, microbial oil, in particular of Stramanopiles, preferably of the class Labyrinthulea, more preferably of the genera Aurantiochytrium or Schizochytrium, and 5 to 95 wt.-%, in particular 10 to 90 wt.-% or 20 to 80 wt.-%, above all 30 to 70 wt.-%, oil of a marine organism, preferably fish oil, or vegetable oil, in particular canola oil or soy oil.

15. Lipid according to claim 13 or 14, wherein the lipid has at least one of the following characteristics:
a) a peroxide value of less than 0.5, preferably less than 0.3, in particular less than 0.15; b) an anisidine value of less than 15, preferably less than 10; c) a TOTOX value of less than 20, preferably less than 15; d) a content of free fatty acids of less than 5 wt.-%, preferably less than 3 wt.-%; e) a content of moisture and impurities of less than 1 wt.-%, preferably less than 0.5 wt.-%; f) a content of omega-3 fatty acids, in particular of DHA and EPA, of at least 10 wt.-%, preferably at least 15 or 20 wt.-%, above all at least 30, 40 or 50 wt.-%; g) preferably DHA and EPA each in an amount of at least 8 wt.-%, preferably at least 10 wt.-%, above all at least 15 wt.-%.; h) preferably an amount of organic solvents of less than 0.5 wt.-%, more preferably less than 0.1 wt.-%, in particular less than 0.05 wt.-%, above all less than 0.01 wt.-%; i) preferably an amount of chlorides of less than 0.1 wt.-%, more preferably less than 0.05 wt.-%, in particular less than 0.01 wt.-%; j) preferably a content of crude fat of more than 90 wt.-%, preferably more than 95 wt.-%.
